# EUROPÄISCHE PATENTANMELDUNG

(11) **EP 1 980 568 A1**
(43) Veröffentlichungstag der Anmeldung: **15.10.2008**
(21) Anmeldenummer: 07007635.1
(22) Anmeldetag: 13.04.2007
(51) Int. Cl.: C07H 21/00, A61K 31/7084, A61P 35/00

(54) **Ethinylierte Heterodinucleosidphosphatanaloga, Verfahren zu deren Herstellung und deren Verwendung**

(71) Anmelder: Eberhard Karls Universität Tübingen, 72076 Tübingen (DE)
(72) Erfinder: Schott, Herbert, 72076 Tübingen (DE); Ludwig, Peter, 72764 Reutlingen (DE)
(74) Vertreter: Schweiger, Georg

(57) **Zusammenfassung**

Es werden neue ethinylierte Heterodinucleosidphosphatanaloga der Fromel I 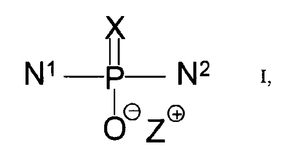 sowie deren Herstellung beschrieben, worin N¹ und N² verschieden sind und beide für ein Nucleosidderivat stehen, von dem mindestens eines der Nucleosidderivate einen Ethinylrest trägt. Die Verbindungen eignen sich zur Behandlung von Krebserkrankungen und Virus bedingten Infektionen.

## Beschreibung

Die vorliegende Erfindung betrifft neue Wirkstoffe, deren Herstellung, Mittel enthaltend wenigstens eine dieser Verbindungen sowie deren Verwendung zur Behandlung von Krebserkrankungen und Infektionskrankheiten.

### Hintergrund der Erfindung

Nucleosidanaloga, die bestimmte Strukturmerkmale aufweisen, sind bewährte Arzneimittel in der Chemotherapie von Krebserkrankungen und virusbedingten Krankheiten (Advanced Drug Delivery Review (1996)19,287). Analoga des Cytidins, wie z.B. 1β-D-Arabinofuranosylcytosin (araC), oder des Uridins, wie z.B. 5-Fluoro-2-deoxyuridin (5FdU), verhindern die DNA-Replikation und wirken gegen maligne Erkrankungen der blutbildenden Zellen und gegen solide Tumore. Zur Therapie der HIV-Infektion eignen sich besonders Didesoxynucleosidanaloga wie z.B. 3'-Azido-2',3'-didesoxythymidin (AZT), 2',3'-Didesoxycytidin (ddC), 2',3'- Didesoxyinosin (ddl), 3'-Thia-2',3'didesoxycytidin (3TC) und 2',3'-Didehydro-2',3'-didesoxythymidin (d4T). Nucleosidanaloga mit Ethinylresten, wie z.B. 3'-C-Ethinylcytidin (ECyd), sind multifunktionelle Antitumor-Wirkstoffe mit einem breiten Aktivitätsspektrum (Hattori, H. et al. J.Med.Chem. 1996, 39, 5005; Azuma, A. et.al. Nucleosides, Nucleotides & Nucleic Acids, 2001, 20. 609)

Die therapeutische Wirkung von Nucleosidanaloga setzt voraus, dass die applizierten Nucleosidanaloga, die in der Regel unwirksame "Prodrugs" sind, von der Zelle aufgenommen und zu den eigentlichen Wirkstoffen, den 5'-Triphosphatderivaten der Nucleosidanaloga anabolisiert werden. Die phosphorylierten Derivate können die DNA- und/oder RNA-Synthese mit lethalen Folgen für die Zelle beeinträchtigen oder die Virusvermehrung unterbinden.

Aufgrund der häufig auftretenden Resistenzbildung im Verlauf der Chemotherapie mit nur einem Wirkstoffe (Monotherapie) kann der applizierte Arzneistoff im Laufe der Therapie seine Wirkung verlieren. Um die Progression der Erkrankung nachhaltig zu verlangsamen und um der Resistenzbildung effektiv entgegenzuwirken, werden verschiedene Wirkstoffe gemeinsam (Kombinationstherapie) appliziert. Die Applikation einer Darreichungsform zur HIV Therapie (Schweiz. Med. Wochenschr. (1997), 127, 436) in der z.B. AZT und 3TC als Gemisch vorliegen, wie dies bei "Combivir" der Fall ist, macht die Kombinationstherapie für den Patienten allenfalls nur praktikabler. Eine verbesserte antivirale Wirkung ist aber mit solchen Gemischen kaum erreichbar, da weder die Zellaufnahme der als Gemisch applizierten Wirkstoffe gesteigert, noch deren Anabolisierung in die entsprechenden Triphosphatderivate optimiert wird.

Dagegen lässt sich die antivirale und/oder cytostatische Wirkung von Kombinationspräparaten erheblich optimieren, wenn die unterschiedlichen Nucleosidanaloga in einer Darreichungsform chemisch gekuppelt sind. Ausschlaggebend für die therapeutische Wirkung solcher Kombinationspräparate ist die jeweils gewählte Art der Verknüpfung der beiden monomeren Wirkstoffe zu einem neuen Kombinationswirkstoff. Die kovalente Kupplung muss gewährleisten, dass bei einer gewünschten Metabolisierung therapeutisch wirksame Metabolite freigesetzt werden können, die additive oder gar synergistische Effekte auslösen und möglicherweise Resistenzmechanismen gegen die monomeren Wirkstoffe aufheben.
Bei der Kupplung eines lipophilen mit einem hydrophilen Nucleosidanaloga über eine Phosphodiesterbrücke resultieren amphiphile Dinucleosidphosphatanaloga (EP-A-0642527). Die Verknüpfung unterschiedlicher hydrophiler Nucleosidanaloga über ein Glycerinlipidgerüst führt zu amphiphilen Glycerylnucleotiden (DE-A-19855963 oder WO-A-00/34298). Die in diesen Kombinationspräparaten gewählte Art der Kupplung erfüllt die gestellte Anforderung. Beide Kombinationspräparate tragen erheblich zur Verbesserung der Chemotherapie von Tumor- und Viruserkrankungen bei.

Das breite Aktivitätssprektrum von Nucleosidanaloga, die einen Ethinylrest aufweisen, wird bisher nur auf der Basis der Glycerylnucleotidanaloga genutzt. Ein deutlicher Nachteil dieser Art von Kombinationspräparaten ist ihr hoher Syntheseaufwand, der vor allem durch die mehrstufige Synthese des Glyceryllipidgerüstes bedingt wird. Außerdem kann das resultierende hohe Molgewicht die Verteilung dieser Kombinationspräparate und damit verbunden das Wirkstofftargeting in vivo erschweren, wodurch die therapeutische Wirkung beeinträchtigt werden kann.

Inwieweit sich eine natürliche oder unnatürliche Phosphodiesterbrücke zur Verknüpfung von ethinylierten Nucleosidanaloga mit anderen therapeutisch wirksamen Verbindung auf Nucleosidbasis eignen, ist weitgehend ungeklärt, da der Einfluss eines Ethinylrestes auf die Metabolisierung eines Dimeren ungeklärt ist.

### Zusammenfassung der Erfindung

Aufgabe dieser Erfindung ist es, neue, leicht zugängliche Kombinationspräparate bereitzustellen, mit denen Krebs- und/oder Viruserkrankungen in neuartiger Weise behandelt werden können.

Die Aufgabe wird überraschenderweise durch neue Dinucleosidphosphatanaloga gelöst, die bei ihrer Metabolisierung gleichzeitig mehrere, verschieden aktive Nucleosidanaloga, wie z.B. mit unterschiedlichen Wirkmechanismen, freisetzen, von denen immer mindestens ein Nucleosidanaloga den therapeutisch hochwirksamen Ethinylrest trägt. Mit diesen so genannten Duplexwirkstoffen werden nicht nur die allgemeinen Vorteile einer Kombinationstherapie, sondern erstmals auch die multifunktionelle Wirksamkeit von ethinylierten Nucleosidanaloga in Kombinationspräparaten nutzbar. Der erforderliche, entscheidende Phosphorylierungsschritt zur Aktivierung der ethinylierten Nucleosidanaloga durch körpereigene Kinasen, wie die Uridin/Cytidin-Kinase, kann bei den Duplexwirkstoffen entfallen, da bei deren Metabolisierung das entinylierte Nucleosid, wie z.B. Ecyd, bereits in der phosphorylierten Form gebildet werden kann. Hinzukommt, dass im Vergleich zu den ethinylierten Glycerylnucleotidanaloga der Syntheseaufwand für die Dinucleosidphosphatanaloga erheblich geringer ist.

Überraschenderweise zeigt außerdem eine erfindungsgemäß bevorzugte Klasse von ethinylierten Dinucleosidphosphatanaloga, die über eine natürliche 3'-5'-Phosphodiesterbrückeoder analoge End-Ring-Verknüpfungen verknüpft sind, und insbesondere solche, die das ethinylierte Monomer über dessen 5'- Position am 3'- Ende des nicht ethinylierten zweiten Monomers tragen, eine signifikant höhere AntitumorAktivität als die entsprechenden Isomere, die eine unnatürliche 5'-5'- Kupplung aufweisen. Die deutliche Überlegenheit der 3'-5'- Kupplung kann anhand der ermittelten Wirkstoffkonzentrationen für die totale Wachstumshemmung der Tumorzellen (siehe Beispiel 3, TGI Werte) gezeigt werden. Häufig liegen die TGI Werte für das 3'-5'- gekuppelte Isomer bis zu 1000-fach niedriger im Vergleich zu den Werten für das 5'-5'-gekuppelte Isomer. Die Art der Verknüpfung ist überraschenderweise für eine weiter verbesserte Wirksamkeit mit ausschlaggebend, da vermutlich bei der enzymatischen Metabolisierung sehr unterschiedlich aktive Wirkstoffe gebildet werden. Durch die Wahl der Richtung der Phosphodiesterbrücke lässt sich die Antitumoraktivität eines Kombinationspräparates überraschenderweise so modulieren, dass das Wirkspektrum der ethinylierten Duplexwirkstoffe weiter verbessert werden kann. Hinzu kommt noch, dass die natürliche 3'-5'-Verknüpfung gegenüber der unnatürlichen 5'-5'- Kupplung synthetisch wesentlich einfacher zugänglich ist und dann bevorzugt ist, wenn die Molekülstruktur der zur kuppelnden Monomere eine natürliche 3'-5'- Phosphodiesterbindung erlaubt.

### Detaillierte Beschreibung der Erfindung

### a) Allgemeine Begriffe

Werden keine anderen Angaben gemacht, so werden erfindungsgemäß sowohl einzelne Isomere erfindungsgemäßer Wirkstoffe als auch jegliche Mischungen stereoisomerer Formen davon mit umfasst. Insbesondere werden alle stereoisomeren Formen erfindungsgemäßer Verbindungen in Reinform sowie jegliche Mischungen solcher stereoisomerer Formen mit umfasst.

Weiterhin können erfindungsgemäße Dinucleosidphosphatanaloga beliebige Kombinationen von D- und L-Isomeren ihrer Nucleosidbausteine umfassen.

Weiterhin sind erfindungsgemäß alle möglichen diastereomeren oder anomeren Formen erfindungsgemäßer Verbindungen, insbesondere alpha- und beta-Anomere, mit umfasst.

Nucleosidreste umfassen erfindungsgemäß natürliche Nucleoside, wie Adenosin, Guanosin, Cytidin, Thymidin, Uridin, Inosin, die korrespondierende Mono- und Didesoxyformen und strukturanaloge Verbindungen, erhältlich durch Änderung des glycosidischen Rests und oder des Basenrests, wie unten näher erläutert.

Die terminale oder endständige Verknüpfung eines Nucleosids erfolgt gewöhnlich über eine HOCH₂- oder HSCH₂-Gruppe, eine ringständige Verknüpfung erfolgt gewöhnlich über eine -CH(OH)- oder -CH(SH)-Gruppe des glycosidischen Rests.

Erfindungsgemäße Verbindungen umfassen je nach Art der ggf. verwendeten Substituenten Verbindungen mit amphiphilen, lipophilen oder hydrophilen Charakter.

Eine erfindungsgemäße Behandlung einer Erkrankung umfasst sowohl die Prophylaxe als auch insbesondere die Therapie.

### b) Erfindungsgemäße Heterodinucleosidphosphatanaloga

Gegenstand der Erfindung sind insbesondere ethinylierte Heterodinucleosidphosphatanaloga der Formel I 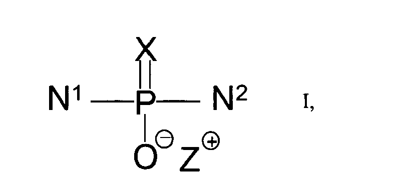 worin
X für O oder S steht;
Z für H oder das korrespondierende Säureadditionssalz dieser Verbindung steht;
N¹ und N² verschieden sind und jeweils für eine Nucleosidgruppe stehen, wobei jede der Nucleosidgruppen, die jeweils einen glycosidischen oder davon abgeleiteten cyclischen Rest und einen damit kovalent verknüpften Basenrest aufweisen, über deren glycosidischen Rest mit dem zentralen P-Atom kovalent verbunden, insbesondere sauerstoff- oder schwefelverknüpft, ist; und wobei wenigstens eine der Nucleosidgruppen einen ethinylierten glycosidischen Rest aufweist. Insbesondere ist die Verknüpfung eine enzymatisch, insbesondere in vitro oder in vivo, im menschlichen Körper spaltbare Verknüpfung. Weiterhin kann die Verknüpfung der glycosidischen Reste terminalterminal (End-End-Verknüpfung, wie z.B. 5'-5') oder terminal-ringständig (End-RingVerknüpfung, wie z.B. 3'-5') erfolgen.

Gegenstand der Erfindung sind insbesondere Verbindungen der Formel 1, wobei jeder der gleichen oder verschiedenen, gegebenenfalls ethinylierten glycosidischen Reste von N¹ und N², der insbesondere als pyranosidischer oder als furanosidischer Rest vorliegt, abgeleitet ist von einer Pentose, Hexose oder Heptose, wobei ein oder mehrere ringgebundene H-Atome oder Hydroxylgruppen gegebenenfalls eliminiert oder substituiert sind durch Halogen, Hydroxyl, Cyano, 2-Fruormethylen, Trifluoromethyl oder Azido; gegebenenfalls ein Heteroatom, ausgewählt unter S, N und O anstelle eines Ring-Kohlenstoffatoms im glycosidischen Rest enthalten sein kann; und der glycosidische Rest gegebenenfalls eine oder zwei nichtbenachbarte C=C-Doppelbindungen enthalten kann.

Gegenstand der Erfindung sind insbesondere auch Verbindungen der Formel 1, wobei jeder der gleichen oder verschiedenen Basenreste der Rest einer ein- oder zweikernigen heterocyclischen Base ist, die aus einem oder zwei vier- bis siebengliedrigen Ringen aufgebaut ist, wobei der Basenrest wenigstens ein basisches Ring-N-atom und gegebenenfalls wenigstens eine basische Aminogruppe und gegebenenfalls wenigstens ein weiteres Ring-Heteroatom, ausgewählt unter S und O, enthalt; und wobei der Basenrest gegebenenfalls ein- oder mehrfach, wie z.B. 1-, 2-, 3-, 4-, 5- oder 6- fach substituiert ist durch Hydroxyl, Amino, Halogen, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio; wobei die Amino-, Alkyl-, Alkenyl- und Acylreste gegebenenfalls mit 1, 2 oder 3 Aryl-Resten, Polyoxyalkylenresten oder Halogenatomen substituiert sind.

### b2) Definitionen allgemeiner Reste

Der glycosidische Rest des Nucleosids oder Nucleosidderivats ist abgeleitet von einer Hexose oder Heptose, vorzugsweise aber von einer Pentose, wie z.B. Desoxyribose, Didesoxyribose oder Ribose. Im glycosidischen Rest können gegebenenfalls einzelne oder mehrere Protonen oder Hydroxylgruppen substituiert oder eliminiert sein. Geeignete Substituenten sind dabei ausgewählt unter Wasserstoff, Halogen, wie F, CI, Br und J, Hydroxyl, Ethinyl, Trifluormethyl, Cyano, 2-Fruormethylen, und Azido. Gegebenenfalls kann ein Heteroatom, ausgewählt unter S, N und O anstelle eines Kohlenstoffatoms enthalten sein und gegebenenfalls kann der Zuckerrest eine oder zwei nichtbenachbarte C=C-Doppelbindungen enthalten.

Der Basenteil des Nucleosids oder Nucleosidderivats ist der Rest einer ein- oder zweikernigen heterocyclische Base, zusammengesetzt aus einem oder zwei vierbis siebengliedrigen Ringen, welche zusammen wenigstens ein Ring-Heteroatom, wie z.B. ein bis sechs Heteroatome, ausgewählt unter N, S und O, insbesondere N und O, enthalten. Beispiele für derartige Basen sind die Purin- und Pyrimidin-Basen Adenin, Guanin, Cytosin, Uracil und Thymin. Weitere Beispiele für brauchbare Basen sind Pyrrol, Pyrazol, Imidazol, Aminopyrazol, 1,2,3-Triazol, 1,2,4-Triazol, Tetrazol, Pentazol, Pyridon, Piperidin, Pyridin, Indol, Isoindol, Pyridazin, Indoxyl, Isatin, Pyrazin, Piperazin, Gramin, Tryptophan, Kynurensäure, Tryptamin, 3-Indolylessigsäure, Carbazol, Indazol, 1,3,5-Triazin, 1,2,4-Triazin, 1,2,3-Triazin und Tetrazin. Bevorzugte Basen sind Adenin, Guanin, Cytosin, Uracil und Thymin; sowie 1,2,3-Triazol, 1,2,4-Triazol und Tetrazol. Die genannten Basen können gegebenenfalls ein- oder mehrfach, wie z.B. ein- bis vierfach, insbesondere ein- oder zweifach, substituiert sein durch die oben angegebenen Reste Hydroxyl, Amino, Halogen, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio, wobei die Alkyl-, Alkenyl- und Acyl gegebenenfalls mit 1 bis 3 Aryl- Resten oder Halogenatomen substituiert sind. Dabei kann die Substitution an einem Ring-Heteroatom oder bevorzugt an einem Ring-Kohlenstoffatom oder einer Seitengruppe, wie z.B. einer Amino-Seitengruppe der Base erfolgen.

Erfindungsgemäße Verbindungen können zudem an einer oder beiden, vorzugsweise einer der Nucleosidgruppen gezielt mit einem lipophilen Rest substituiert sein. Der lipophile Rest sollte ein geradkettiger oder verzweigter Kohlenwasserstoffrest, insbesondere Alkyl-, Alkenyl-, Acyl-, Alkyloxy-, Acyloxy,- Aryloxy-, Alkenyloxy-, Alkylthio-, Alkenylthio-, Acylthio- oder Arylthio-Rest, wie im folgenden definiert, sein und vorzugsweise mehr als 6, wie z.B. 7 bis 30 oder 10 bis 24 Kohlenstoffatome umfassen.

Als Beispiele für geeignete Aryl-Reste sind zu nennen: Phenyl, Naphthyl, und Benzyl.

Als Beispiele für geeignete Alkylreste sind zu nennen geradkettige oder verzweigte Reste mit 1 bis 24 C-Atomen, wie Methyl, Ethyl, i- oder n-Propyl, n-, i-, sec.- oder tert.-Butyl, n- oder i-Pentyl; außerdem n-Hexyl, n-Heptyl, n-Octyl, n-Nonyl, n-Decyl, n-Undecyl, n-Tridecyl, n-Tetradecyl, n-Pentadecyl und n-Hexadecyl, Octadecyl, Docosanyl, sowie die ein- oder mehrfach verzweigten Analoga davon.

Als Beispiele für geeignete Alkenylreste sind die ein- oder mehrfach, vorzugsweise einfach oder zweifach, ungesättigten Analoga oben genannter Alkylreste mit 2 bis 24 Kohlenstoffatomen, wobei die Doppelbindung in beliebiger Position der Kohlenstoffkette liegen kann.

Beispiele für geeignete Polyoxyalkenylreste sind abgeleitet von C₂-C₄-Alkylenoxiden, welche 2 bis 12 wiederkehrende Alkyenoxideinheiten umfassen können.

Beispiele für geeignete Acylreste sind abgeleitet von geradkettigen oder verzweigten, gegebenenfalls ein- oder mehrfach ungesättigten, gegebenenfalls substituierten C₁-C₂₄-Monocarbonsäuren. Beispielsweise sind brauchbare Acylreste abgeleitet von folgenden Carbonsäuren: Gesättigten Säuren , wie Ameisen-, Essig-, Propion- und n- und i-Buttersäure, n- und i-Valeriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure; einfach ungesättigte Säuren, wie Acrylsäure, Crotonsäure, Palmitoleinsäure, Ölsäure und Erucasäure; und zweifach ungesättigten Säuren, wie Sorbinsäure und Linolsäure. Sind in den Fettsäuren Doppelbindungen enthalten, so können diese sowohl in cis- als auch in trans-Form vorliegen.

Beispiele für geeignete Alkyloxy-, Acyloxy,- Aryloxy- Alkenyloxy und Polyoxyalkylenoxyreste sind die Sauerstoff-terminierten Analoga oben genannter Alkyl-, Acyl-, Aryl-, Alkenyl und Polyoxyalkylenreste.

Beispiele für geeignete Alkylthio-, Alkenylthio-, Acylthio- oder Arylthio-Reste sind die entsprechenden Schwefel-terminierten Analoga obiger Alkyloxy-, Alkenyloxy-Acyloxy- und Aryloxy-Reste.

### b3) Bevorzugte Verbindungen

Gegenstand der Erfindung sind insbesondere auch Verbindungen der Formel 1, wobei N¹ und N² über gleiche oder verschiedene Positionen der glycosidischen Gruppen P-verknüpft sind.

Gegenstand der Erfindung sind insbesondere auch Verbindungen der Formel 1, wobei jeder der gleichen oder verschiedenen glycosidischen Reste ein furanosidischer Rest oder ein davon abgeleiteter, fünfgliedriger Rest ist; insbesondere sind dabei N¹ und N² über das P-Atom 3'-5' oder 5'-5' miteinander verknüpft sind.

Insbesondere sind solche Verbindungen bevorzugt, worin der terminal verknüpfte, insbesondere der 5'-verknüpfte furanosidische Rest des einen Nucleosids die Ethinylierung trägt, und zwar insbesondere in 3'- oder 2'-Position des furanosidischen Rings. Der Ring-verknüpfte, insbesondere 3'-verknüpfte, furanosidische Rest des anderen Nucleosids ist dagegen nicht ethinyliert.

Gegenstand der Erfindung sind insbesondere auch Verbindungen der Formel 1, worin X und Z die oben angegebenen Bedeutungen besitzen, Y Sauerstoff oder Schwefel bedeuten und die Gruppen N¹ und N² voneinander verschieden sind und für ein D- oder L- konfiguriertes Nucleosidderivat der Formel II, III, und IV 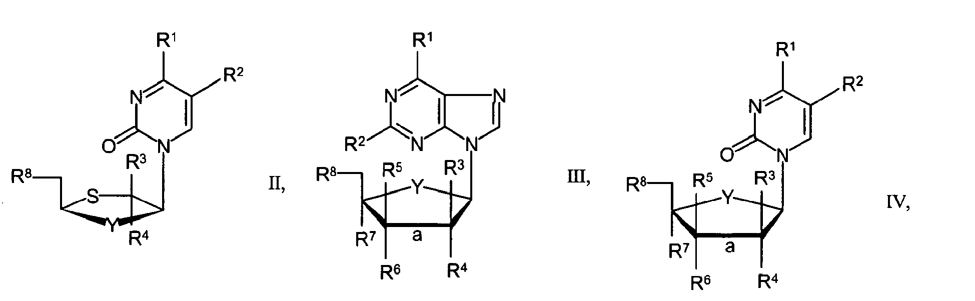 stehen, worin
Y für O oder S steht;
R¹ eine Hydroxyl-, Alkoxy-, Amino-, acylierte, alkylierte oder durch Polyoxyethylen substituierte Aminogruppe bedeutet, deren Acyl- oder Alkylrest linear oder verzweigt ist, 1 bis 24 C-Atome und bis zu 2 Doppelbindungen aufweist und durch 1, 2 oder 3 aromatische Reste oder einen Heterocyclus substituiert sein kann,
R² für H, Halogen, eine Amino-, Hydroxyl- oder Trifluormethylgruppe, einen Bromvinyl-, einen linearen oder verzweigten C₁-C₂₄ Alkylrest steht;
R³ bis R⁸ gleich oder verschieden sind, und für H, Halogen, Hydroxyl, Ethinyl, Cyano, Fluoromethylen, Trifluoromethyl oder Azido stehen, wobei zwei der Reste R³ bis R⁶ dann entfallen, wenn die C-C-Bindung in Position a für eine Doppelbindung steht;
wobei N¹ und N² so gewählt sind, das immer einer der Reste R³ bis R⁸ von N¹ und N² unabhängig voneinander für -O- oder -S- steht, worüber N¹ und N² mit dem zentralen P-Atom der Formel I verknüpft sind; und mindestens einer der verbleibenden Reste R³ bis R⁸ in N¹ oder N² Ethinyl bedeutet, so dass entweder N¹ oder N² mindestens einen Ethinylrest aufweist.

Gegenstand der Erfindung sind insbesondere auch Verbindungen der Formel I, worin X, Y, Z und a die oben angegebenen Bedeutungen besitzen und
a) in den Gruppen N¹ und N² unabhängig voneinander
   R¹ für eine alkylierte oder acylierte Aminogruppe steht, worin deren Alkylrest ein Hexadecylrest und der Acylrest ein Palmitoyl, Oleoyl- oder Behenoylrest ist;
   R² für H, Halogen, Methyl, Ethyl oder Trifluormethyl steht;
   R³, R⁴ und R⁷ für Azido, H, Fluor, Fluoromethylen, Cyano, Trifluoromethyl oder Hydroxyl steht;
      und gleichzeitig
b) in einer der Gruppen N¹ und N²
   R⁵ für Ethinyl steht und in der anderen der Gruppen R⁵, falls vorhanden, für Azido, H, Fluor oder Hydroxyl steht;
   und gleichzeitig
c) in jeder der Gruppen N¹ und N² unabhängig voneinander
   einer der Reste R⁶ und R⁸ für -O- oder -S- steht und der andere der beiden Reste R⁶ und R⁸ für Azido, H, Fluor oder Hydroxyl steht.

Weitere Gruppen bevorzugter Verbindungen sind:
(1) Verbindungen
   worin Z und a die oben angegebenen Bedeutungen besitzen
   X für O steht;
   N¹ und N² verschieden sind und für ein Nucleosidderivat der Formel IV stehen,
   worin Y für O steht Sauerstoff;
   R¹ für eine Amino-, C₁₂-C₂₂-Alkylamino-, C₁₂-C₂₂-Acyl-Aminogruppe oder eine
   Hydroxylgruppe steht;
   R² für H, Fluor oder Trifluormethyl steht; und
   R³ bis R⁸ die oben angegebenen Bedeutungen besitzen.
(2) Verbindungen, worin N¹ für einen, vorzugsweise nicht ethinylierten Nucleosidrest der Formel IV steht, worin die Reste R¹ bis R⁸ in die oben angegebenen Bedeutungen besitzen und N² für einen Nukleosidrest der Formel IV steht der ethinyliert ist, worin
   R¹ für Amino
   R²,R³,R⁷ für H;
   R⁴, R⁶ für Hydroxyl;
   R⁵ für Ethinyl; und
   R⁸ für ein Sauerstoffatom steht, mit dem N² verbrückt ist.
(3) Verbindungen, worin N² für einen Nukleosidrest der Formel IV steht der ethinyliert ist, und N¹ für einen Nukleosidrest der Formel IV steht, der nicht ethinyliert ist,
   worin
   R¹ für Hexadecyl-, Palmitoyl-, Oleoylamino oder Hydroxyl steht;
   R² für H oder Fluor steht;
   R³, R⁴ gleich oder verschieden sind und für H, Hydroxyl, Fluor steht;
   R⁵, R⁷ für H steht;
   R⁶ und R⁸ unabhängig voneinander für Hydroxyl, Azido oder H stehen, mit der
   Maßgabe, dass einer der Reste für -O- steht.
(4) Verbindungen, worin in N² für einen Nukleosidrest der Formel IV steht der ethinyliert ist, und N¹ für einen Nukleosidrest der Formel IV steht, der nicht ethinyliert ist, worin
   R¹ für Hydroxyl steht;
   R² für Fluor steht;
   R³, R⁴, R⁷ für H stehen,
   R⁶ für -O- steht, und
   R⁸ für Hydroxyl steht.

Bevorzugte Klassen von heterodinucleosidphosphatanaloga umfassen insbesondere als ethinylierte Komponente einen Nucleosidrest von einem 2'-, 3' oder 4'-C-Ethinylnucleosid, wie insbesondere von 2'-, 3' oder 4'-C-Ethinylcytidin oder 2'-, 3' oder 4'-C-Ethinyluridin, insbesondere 2-, oder 3'-C-Ethinylcytidin.

Bevorzugte Einzelverbindungen sind ausgewählt unter:
5-Fluoro-2'-desoxyuridylyl-(3'-5')-3'-C-ethinylcytidin
Arabinocytidylyl-(5'- 5')-3'-C-ethinylcytidin
N⁴-Hexadecylarabinocytidylyl-(5'- 5')-3'-C-ethinylcytidin
(E)-2'-Desoxy-(2-fluoromethylen)cytidyly-(3' 5')-3'-C-ethinylcytidin
β-L-Dioxolancytidylyl-(5'- 5')-3'-C-ethinylcytidin
2'-C-Cyano-2'-deoxyarabinocytidylyl-(3'-5')-3'-C-ethinylcytidin
2-Chloro-(2'-deoxy)-fluoroarabinoadenylyl-(3'-5')-3'-C-ethinylcytidin
2'-Desoxy-2',2'-difluorocytidylyl-(3'-5')-3'-C-ethinylcytidin

### c) Herstellung erfindungsgemäßer Heterodinucleosidphosphatanaloga

Die Erfindung betrifft außerdem Verfahren zur Herstellung von erfindungsgemäßen ethinylierten Heterodinucleosidphosphatanaloga, wobei man zwei Nucleoside der allgemeinen Formeln Va und Vb

L¹-N¹ (Va)

L²-N² (Vb)

worin
N¹ und N² wie oben definiert sind und gegebenenfalls eine oder mehrere Schutzgruppen aufweisen, und
L¹ und L² am glycosidischen Rest von N¹ und N² gebundene, miteinander reaktive Gruppen darstellen, wobei eine der Gruppen L¹ und L² für eine Hydroxy- oder Mercaptogruppe und der andere für eine Hydrogenphosphonat- oder Thiohydrogenphosphonatgruppe steht;
in Gegenwart eines Säurechlorids kondensiert und das Kondensationsprodukt anschließend oxidiert, und gegebenenfalls vorhandenen Schutzgruppen entfernt.

Gegenstand ist insbesondere ein Herstellungsverfahren, wobei man jeweils zwei Nucleoside der Formeln Va und Vb umsetzt, wobei die Nucleoside Va und Vb einer Verbindung der obigen allgemeinen Formel II, III oder IV entsprechen, worin
X und a die oben angegebenen Bedeutungen besitzen,
L¹ und L² an Stelle eines der Reste R³ bis R⁸ enthalten sind, die Reste R¹ bis R⁸ im übrigen die in den Ansprüchen 7 bis 17 angegebene Bedeutung
besitzen;
die Reste R¹ und R³ bis R⁸ zusätzlich auch für eine acylierte Hydroxygruppe, deren Acylrest linear oder verzweigt ist, 1-24 C-Atome und 1 oder 2 Doppelbindungen aufweist und durch einen aromatischen Rest substituiert sein kann, oder für tert-Butyldimethylsilyloxy-Schutzgruppe stehen können,
R⁸ zusätzlich auch für eine 4-Mono-, oder 4,4'-Dimethoxytriphenylmethyloxy-Schutzgruppe stehen kann;
und wenigstens einer der Reste R³ bis R⁸ auch für Trimethylsilylethinyl stehen kann.

Weiterhin ist dabei bevorzugt, dass man gegebenenfalls vorhandene 4-Mono- oder 4,4'- Dimethoxytriphenylmethyloxy Schutzgruppen gegen Hydroxyl tauscht und Acyl- und Silylreste gegebenenfalls hydrolytisch spaltet.

Werden keine anderen Angaben gemacht, so erfolgt die Herstellung erfindungsgemäßer Verbindungen unter Anwendung an sich bekannter Verfahren oder Synthesetechniken die dem Fachmann auf dem Gebiet der Synthese von Nucleosidanalogen geläufig sind.

Die Kondensation gelingt besonders gut in Lösung in Gegenwart von Säureanhydriden oder Säurehalogeniden, wie insbesondere Pivalinsäurechlorid, bei -80 °C bis +100°C, wie z.B. etwa 0 - 20°C. Ein geeignetes Lösungsmittel ist z.B. Pyridin.

Die Oxidation gelingt besonders gut in Lösung bei -80 °C bis +100 °C, wie z.B. etwa 0 - 20°C, wobei, a) mit Jod in wässrigen organischen Lösungsmitteln die P-H-Bindung zu einer P=O-Bindung oder b) mit S₈ in Triethylamin/CS₂ die P-H-Bindung zu einer P=S-Bindung oxidiert wird. Ein geeignetes Lösungsmittel ist z.B. THF.

Nach Oxidation und chromatographischer Aufarbeitung erfolgt die Abtrennung der Schutzgruppen in an sich bekannter Weise. Dabei wird beispielsweise die 4-Mono- oder 4,4'-Dimethoxytriphenylmethylgruppe gegen Hydroxyl, und/oder Trimethylsilyl gegen Wasserstoff getauscht und/oder Acylreste werden im Bedarfsfall hydrolytisch in Mercapto-, Hydroxyl- und/oder Aminogruppen überführt.

Die für die Umsetzungen benötigten Ausgangsmaterialien sind an sich bekannte Stoffe oder lassen sich in Analogie zu bekannten Verfahren herstellen (Antivir. Chem & Chemother. (1998) 9, 33; Makromol Chem. (1986) 187, 809; Tetrahedron Lett. (1986) 27, 2661; Synthesis (2002) 16, 2387; Eurp. J Med.Chem. (2005) 40, 494).

Insbesondere sind auch die erfindungsgemäß verwendeten ethinylierten Nucleosidbausteine an sich bekannt bzw. leicht herstellbar.(vgl. z.B. auch Bioorg. Med. Chem. (2005) 13, 2597-2621; Cancer Sci (2005), 96, 5, 295-302; J. Med. Chem. (1996) 39, 5005-5011; Radiation Research (2004) 162, 635-645).

### d) Erfindungsgemäße pharmazeutische Formulierungen und Anwendungen

Die Kupplung des ethinylierten Nucleosids, wie des therapeutisch hochwirksamen Ethinylcytidins, an ein zweites, ebenfalls wirksames Nucleosidanalogon, resultiert in einem sogenannten Duplex-Wirkstoff, der additive und/oder synergistische Wirkmechanismen auslöst. Dieser Effekt ist dann besonders stark ausgeprägt, wenn beide Monomere unterschiedliche Targets angreifen. Hierdurch kann die applizierte therapeutische Menge der hochpotenten Duplexwirkstoffe im Vergleich zu der für die jeweiligen Monomere so dosiert werden, dass die gewünschte therapeutische Wirkung optimiert, wobei gleichzeitig die unerwünschten toxischen Nebenwirkungen maßgeblich reduziert werden.

Die unterschiedliche Sequenz, eine natürliche 3'-5' oder unnatürliche 5'-5' Phosphodiesterbrücke, in der beide Nucleosidanaloga zum Heterodinucleosid- phosphatanaloga gekuppelt werden können, führt bei der enzymatischen Metabolisierung in vivo zu sehr unterschiedlich, vorausbestimmbaren derivatisierten Metaboliten. Hierdurch kann das therapeutische Spektrum quasi programmiert und maßgebend erweitert werden. Eine Folge davon ist, dass Duplexwirkstoffe bei Resistenzen wirksam sind, gegen die sich die jeweiligen monomeren Nucleosidanaloga als unwirksam erweisen.

Die Überführung von ethinylierten Nucleosiden, wie Ethinylcytidin, in Duplex-Wirkstoffe bedingt auch ein stark verändertes pharmakokinetisches Verhalten, was wiederum zur Optimierung der Therapie beiträgt. Durch die große Variabilität der Derivatisierung sind ethinylierte Duplex-Wirkstoffe je nach der Art der eingeführten Substituenten mit sehr unterschiedlichen Lösungseigenschaften darstellbar. Hierdurch eröffnen sich zahlreiche Möglichkeiten der pharmazeutischen Formulierung, die für das monomere Ethinylcytidin, aufgrund dessen Hydrophilie nicht anwendbar sind.

Ein weiterer Vorteil der erfindungsgemäßen Duplex-Wirkstoffe liegt darin, dass sie zusammen mit einem oder mehreren anderen Wirkstoffen in unterschiedlichen Mengen in Liposomen oder Nanopartikeln eingebaut werden können, wobei synergistische Wirkungen resultieren.

Gegenstand der Erfindung sind damit auch pharmazeutische Mittel, enthaltend mindestens eine Verbindung nach obiger Definition in einem pharmazeutisch verträglichen Träger oder Verdünnungsmittel, wie insbesondere enthalten in Liposomen oder Nanopartikeln.

Weiterhin können erfindungsgemäße Mittel zusätzlich wenigstens einen weiteren pharmakologischen Wirkstoff enthalten, der zur Behandlung von Infektionskrankheiten und/oder Krebserkrankungen geeignet ist.

Gegenstand der Erfindung ist insbesondere auch Verwendung wenigstens einer Verbindung nach obiger Definition zur Herstellung eines pharmazeutischen Mittels zur Prävention und/oder Therapie von Infektionskrankheiten und/oder Krebserkrankungen.

Die erfindungsgemäßen Verbindungen werden im allgemeinen in Form pharmazeutischer Mittel zur Behandlung eines Individuums, vorzugsweise eines Säugers, insbesondere eines Menschen, eingesetzt. So werden die Verbindungen gewöhnlich in Form von pharmazeutischen Zusammensetzungen verabreicht, die einen pharmazeutisch verträglichen Exzipienten mit wenigstens einem erfindungsgemäßen ethinylierten Nucleosidphosphatanalogon, gegebenenfalls auch ein Gemisch mehrerer erfindungsgemäßer Verbindungen, und gegebenenfalls weitere für den jeweiligen gewünschten therapeutischen Effekt brauchbare Wirkstoffe umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, Pastillen, Sachets, Cachets, Dragees, Kapseln wie Hart- und Weichgelatinekapseln, Suppositorien oder vaginale Arzneiformen; halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Verbindungen verwendet werden. Ferner können auch Liposomen, Mikrosphären oder Polymermatrizes zur Anwendung kommen.

Bei der Herstellung der Mittel werden erfindungsgemäße Verbindungen gewöhnlich mit einem Exzipienten vermischt oder verdünnt. Exzipienten können feste, halbfeste oder flüssige Materialien sein, die als Vehikel, Träger oder Medium für den Wirkstoff dienen.

Zu geeigneten Exzipienten gehören beispielsweise Lactose, Dextrose, Succrose, Sorbitol, Manitol, Stärken, Akaziengummi, Calciumphosphat, Alginate, Traganth, Gelantine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Bevorzugte übliche Träger sind beispielsweise Mannit, Glucose, Dextrose, Albumine oder ähnliches; bevorzugte Verdünnungsmittel sind im wesentlichen physiologische Kochsalzlösungen oder eine 5%ige Glucoselösung. Weiterhin ist es üblich, derartige Lösungen mit geeigneten Reagenzien, beispielsweise Phosphaten, abzupuffern.

Für eine bessere Applikation der erfindungsgemäßen Verbindungen können Zusammensetzungen bereitgestellt werden, welche die erfindungsgemäßen Verbindungen in Kombination mit einem organischen Träger enthalten. Darüber hinaus können alle weiteren, für die Zubereitung von pharmazeutischen Mitteln üblichen Excipienten hinzugesetzt werden, vorausgesetzt, sie der bestimmungsgemäße Gebrauch derartiger Zusammensetzung aus dem organischen Träger und den erfindungsgemäßen Verbindungen wird nicht beeinträchtigt.

Eine bevorzugte Ausführungsform solcher Zusammensetzungen sieht die Assoziation der erfindungsgemäßen Verbindungen in Form von uni- bis oligolamellaren Liposomen mit einem Durchmesser von maximal 0.4 µm vor. Zur Liposomenbildung können alle an sich bekannten Verfahren der Liposomendarstellung verwendet werden, wie beispielsweise Ultraschall, Gelchromatographie, Detergenzanalyse, Hochdruckfiltration. Die jeweils eingeführten lipophilen Reste beeinflussen maßgeblich die Größe und Stabilität der Liposomen, die sich aus den jeweiligen Glycerylnucleotiden zusammen mit weiteren Lipidkomponenten bilden (vgl. auch Liposomes: From Physical Structure to Therapeutic Applications in: Research monographs in cell and tissue physiology Bd.7, G.G. Knight Hrsg., Elsevier (1981).

Eine weitere bevorzugte Möglichkeit, die erfindungsgemäßen Verbindungen mit einem organischen Träger zu verbinden, ist der Einschluss der Verbindungen in biologisch verträgliche Nanopartikel. Als Nanopartikel werden organisch-chemische Polymere bezeichnet, denen bei der Polymerisation die erfindungsgemäßen Verbindungen zugesetzt werden, so dass sie mit einer gewissen Effizienz in das Nanopartikel eingeschlossen werden (vgl. Bender et al., Antimicrobial agents and Chemotherapy (1996), 40 (6) 1467-1471).

In einer bevorzugten Ausführungsform kann die erfindungsgemäße Zusammensetzung weitere Komponenten umfassen, die eine spezifische Anreicherung im Bereich der zu therapierenden Zellen und/oder Organen begünstigen. Dabei kann beispielsweise die Zusammensetzung der Liposomen so gewählt werden, dass die Liposomen zusätzlich mit Molekülen, wie z.B. Antikörpern, geladenen Lipiden, oder mit hydrophilen Kopfgruppen modifizierten Lipiden versehen werden, damit die Zusammensetzung sich bevorzugt in den zu therapierenden Zellen und/oder Organen oder in deren Umgebung anreichert. Eine solche Zusammensetzung mit spezifisch gegen Tumorzellen, virusinfizierten Zellen und/oder Organe gerichteten Molekülen erhöht die therapeutische Wirkung der Arzneimittel und reduziert gleichzeitig die Toxizität für nicht infizierte Gewebe.

Derartige Zusammensetzungen können zu einem pharmazeutischen Mittel verarbeitet werden, das neben den erfindungsgemäßen Verbindungen und gegebenenfalls dem organischen Träger weiterhin übliche Träger- und/oder Verdünnungsmittel und/oder Hilfsstoffe enthält. Übliche Trägermittel sind beispielsweise Mannit, Glucose, Dextrose, Albumine oder ähnliches, während als Verdünnungsmittel im wesentlichen physiologische Kochsalzlösungen oder eine 5%ige Glucoselösung dient. Weiterhin ist es üblich, die Lösungen mit geeigneten Reagenzien, beispielsweise Phosphaten, abzupuffern. Darüber hinaus können alle weiteren, für die Zubereitung von pharmazeutischen Mitteln üblichen Zusatzstoffe hinzugesetzt werden, vorausgesetzt, die Zusammensetzung aus dem organischen Träger und den erfindungsgemäßen Verbindungen wird nicht negativ beeinflusst.

In einer bevorzugten Ausführungsform können die erfindungsgemäßen Wirkstoffe mit Komponenten kombiniert werden, die sich in den zu therapierenden Zellen und/oder Organen spezifisch anreichern. Dabei kann beispielsweise die Zusammensetzung der Liposomen so gewählt werden, dass die Liposomen zusätzlich mit Molekülen, wie z.B. Antikörper, geladenen Lipiden, mit hydrophilen Kopfgruppen modifizierten Lipiden, versehen werden, damit die Zusammensetzung sich bevorzugt in den zu therapierenden Zellen und/oder Organen anreichert. Eine solche Zusammensetzung mit spezifisch gegen Tumorzellen, virusinfizierten Zellen und/oder Organe gerichteten Molekülen erhöht die therapeutische Wirkung der erfindungsgemäßen Mittel und reduziert gleichzeitig die Toxizität für nicht infizierte Gewebe.

Durch die Überführung in Duplex-Wirkstoffe lassen sich aber nicht nur die enzymatische Hydrolysebeständigkeit erhöhen und die Applikationsformen deutlich erweitern, sondern überraschenderweise auch zytostatische und virusstatische Wirkungen optimieren.

Die Duplex-Wirkstoffe lassen sich gegen maligne Erkrankungen der blutbildenden Zellen und solide Tumore einsetzen. Durch die verbesserte zytostatische Wirkung treten sehr viel weniger gravierende Nebenwirkungen auf. Es können höhere Dosen der zytostatisch wirksamen erfindungsgemäßen Verbindungen eingesetzt werden und es kann in zeitlichen Intervallen therapiert werden.

Insbesondere sind die erfindungsgemäßen Verbindungen einsetzbar zur Prophylaxe und/oder Therapie folgender Tumorerkrankungen: Leukämie, Lungenkrebs, Darmkrebs, Krebs des Zentralnervensystems, Melanomen, Ovarialkrebs, Nierenkrebs, Prostatakrebs und Brustkrebs.

Insbesondere sind die erfindungsgemäßen Verbindungen einsetzbar zur prophylaxe und/oder Therapie folgender Viruserkrankungen: AIDS (HIV-Infektion), Hepatitis A, B und C, Herpes und CMV-Infektionen

Überraschenderweise zeigen die erfindungsgemäßen Duplex-Wirkstoffe auch virusstatische Wirkungen, so dass sie in der Chemotherapie von virusbedingten Infektionen und zur Überwindung von Arzneimittelresistenzen verwendbar sind.

### Beispiel 1

Darstellung von 5-Fluoro-2'-desoxyuridylyl-(3'-5')-3'-C-ethinylcytidin

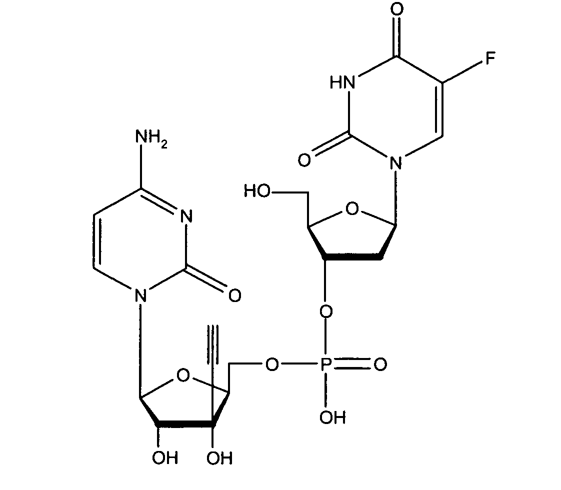

### a) Herstellung des Ausgangsmaterials

19.2 g (33 mmol) 5'-O-(4-Monomethoxytrityl)-5-fluoro-2'-desoxyuridin-3'-phosphonat werden zusammen mit 18.4 g (33 mmol) N⁴-Benzoyl-2'-O-(tert.-butyldimethylsilyl)-3'-C-(trimethylsilylethinyl)cytidin in 100 ml wasserfreiem Pyridin gelöst. Die auf ca. 10°C abgekühlte Lösung wird unter Feuchtigkeitsausschluss mit 24 ml (195 mmol) Pivaloylchlorid versetzt, 5 min. bei Raumtemperatur kräftig geschüttelt, dann schnell auf ca. 0°C gekühlt und mit 20 ml Wasser versetzt. Der Reaktionsansatz wird bei Raumtemperatur einige Minuten gerührt, dann mit 160 ml einer Lösung von 25.4 g lod in 450 ml Tetrahydrofuran versetzt und anschließend 1 h bei Raumtemperatur gerührt. Überschüssiges lod wird durch Zugabe von festem NaHSO₃ reduziert, bevor der Reaktionsansatz im Rotationsverdampfer zum Sirup konzentriert wird, der dann in 300 ml eines Gemisches aus Chloroform/Methanol (9:1) aufgenommen und gegen ca. 250 ml Wasser ausgeschüttelt wird. Die organische Phase wird am Rotationsverdampfer zum Sirup konzentriert, der mit 300 ml Chloroform verdünnt und dann an einer Kieselgelsäule mit einem Chloroform/Methanol-Gradienten fraktioniert wird, dessen Methanolanteil steigend ist. Im Verlauf der Chromatographie wird bereits die Monomethoxytritylgruppe bei einem Teil des Produkts gegen Wasserstoff getauscht. Die Fraktionen, die das Produkt mit und ohne Monomethoxytritylrest enthalten ergeben nach der Konzentration am Rotationsverdampfer ca. 32 g Schaum. Zum vollständigen Austausch der Monomethoxytritygruppe gegen Wasserstoff wird der erhaltene Schaum zusammen mit 50 ml Methanol in 60 ml 80%iger Essigsäure 24h bei Raumtemperatur gerührt und anschließend am Rotationsverdampfer wieder zum Schaum konzentriert. Der Schaum wird mit ca. 120 ml Ether versetzt, kräftig geschüttelt und hierbei in einen Niederschlag überführt, der nach dem Abzentrifugieren und Trockene ca. 25 g Feststoff ergibt. Der Feststoff wird in ca. 250 ml Chloroform gelöst und an einer Kieselgelsäule mit einem Chloroform/Methanol-Gradienten fraktioniert, dessen Methanolanteil steigend ist. Produktenthaltene Fraktionen werden am Rotationsverdampfer zum Schaum konzentriert, der bei Etherzugabe in einen Feststoff übergeht, der nach dem Trocknen 19 g ergibt.

### b) Herstellung des Endprodukts

Der gemäß a) erhaltene Feststoff wird zum Austausch der Silylgruppen gegen Wasserstoff in 170 ml trockenem Tetrahydrofuran gelöst, mit 85 ml einer 1M Lösung von Tetrabutylammoniumfluorid-Trihydrat in Tetrahydrofuran versetzt, verschlossen 3 Tage bei Raumtemperatur gerührt und dann am Rotationsverdampfer zum Sirup konzentriert. Zum anschließenden Austausch des Benzoylrestes gegen Wasserstoff wird der erhaltene Sirup mit ca. 300 ml einer 33%igen Ammoniaklösung versetzt und verschlossen 5 Tage bei Raumtemperatur gerührt. Der Ansatz wird dann am Rotationsverdampfer auf ca. 250 ml konzentriert. Der ausgefallene feine Niederschlag wird abzentrifugiert. Der Überstand wird lyophilisiert. Das Lyophilisat wird in ca. 60 ml Wasser gelöst und an einer präparativen RP-18 Säule mit einem Wasser/Methanol-Gradienten fraktioniert, dessen Methanolanteil steigend ist. Die produktenthaltenen Fraktionen, die bei einem Methanolanteil des Gradienten von ca. 15%-40% die Säule verlassen, werden vereinigt, mit einem Kationenaustauscher (H⁺-Form) auf pH ca. 5.8 eingestellt und vom Austauscher abgetrennt. Das Filtrat wird mit Ammoniak neutralisiert, anschließend konzentriert und dann lyophilisiert. Hierbei werden 10,6 g des gewünschten Produkts als Tetrabutylammoniumsalz erhalten. Die berechneten Molekülmassen für die anionische Form 574.40 und die Tetrabutylammoniumsalzform 815.8 werden im FAB-Massenspektrum durch die Molekülpeaks 574.0 und 815.8 [M-H]- bestätigt.

### Beispiel 2

Darstellung von 5-Fluoro-2'-desoxyuridylyl-(5'-5')-3'-C-ethinylcytidin

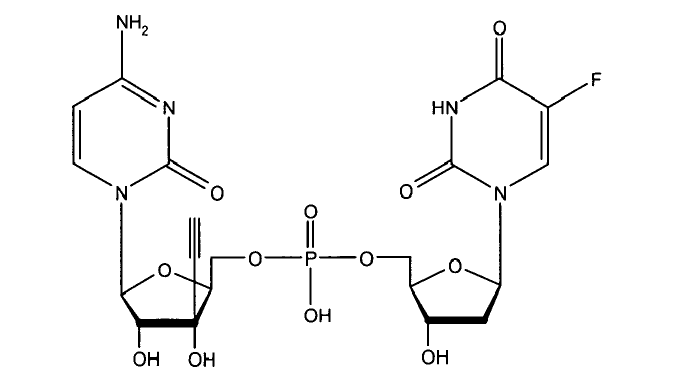

### a) Herstellung des Ausgangsmaterials

11.0 g (21.2 mmol) 3'-4- Di-O-benzoyl-5-fluoro-2'-desoxyuridin-5'-hydrogenphosphonat werden zusammen mit 9.5 g (17 mmol) N⁴-Benoyl-2'-O-(tert.-butyldimethylsilyl)-3'-C-(trimethylsilylethinyl)cytidin in ca. 100 ml wasserfreiem Pyridin gelöst. In Analogie zu a) aus Beispiel 1 wird durch Zusatz von 13 ml (10,6 mmol) Pivaloylchlorid die Kondensation gestartet, die Reaktion nach 5 min. durch Zugabe von 10 ml Wasser abgebrochen und das Kondensat anschließend mit 82 ml einer Lösung von 25,4 g lod in 450 ml Tetrahydrofuran oxidiert. Nach der Aufarbeitung des Reaktionsansatzes, der chromatographischen Reinigung an einer Kieselgelsäule und der anschließenden Etherfällung werden 13 g eines Feststoffes erhalten

### b) Herstellung des Endprodukts

In dem gemäß a) erhaltenen Feststoff werden in Analogie zu b) aus Beispiel 1 zunächst die Silylgruppen gegen Wasserstoff getauscht, indem der Feststoff mit 45 ml Tetrahydrofuran und 22 ml eine 1 M Lösung von Tetrabutyammoniumfluorid-Trihydrat in Tetrahydrofuran 3 Tage bei Raumtemperatur behandelt und dann zum Sirup konzentriert wird. Anschließend wird der Sirup zum Austausch der Benzoylreste gegen Wasserstoff in 80 ml eine 33%igen Ammoniaklösung 5 Tage verschlossen gerührt. Der aufgearbeitete Reaktionsansatz wird lyophilisiert und das erhaltene Lyophilisat wie unter b) im Beispiel 1 beschrieben an einer präparativen RP-18 Säule fraktioniert. Die Produktfraktionen werden in 5,8 g eines Lyophilisats überführt. Die berechneten Molekülmassen für die anionische Form 574.40 und die Tetrabutylammoniumsalzform 815.8 werden im FAB-Massenspektrum durch die Molekülpeaks 574.0 und 815.8 [M-H]- bestätigt.

### Beispiel 3

Die in vitro zytostatische Wirkung der erfindungsgemäßen Verbindungen läßt sich durch folgende Versuchsanordnung zeigen.

Als Testsystem dienen Tumorzellinien, deren 100%ige (TGI) Wachstumshemmung durch die erfindungsgemäßen Verbindungen bei verschiedenen Konzentrationen ermittelt wird. Ferner wird die Toxizität (LC₅₀) der Verbindung auf diese Zellen ermittelt. Eine Serie von Mikrotiterplatten wird am Tag 0 mit den Tumorzellen beimpft und für 24 h vorinkubiert. Danach wird die erfindungsgemäße Verbindung den Zellen in fünf jeweils 10-fach verdünnter Konzentration zugegeben, ausgehend von der höchsten löslichen Konzentration. Es folgt eine 48.stündige Inkubation, an deren Ende die Zellen in situ fixiert, gewaschen und getrocknet werden. Anschließend wird Sulforhodamin B(SRB), ein pinker Farbstoff der an die fixierten Zellen gebunden wird, zugefügt und die Zellen erneut gewaschen. Der verbleibende Farbstoff spiegelt die adhärente Zellmasse wieder und wird spektroskopisch bestimmt. Die automatisch erfassten Daten werden per Computer ausgewertet und führen für die erfindungsgemäße Verbindung 5-Fluoro-2'-deoxyuridylyl-(5'-5')-3'-C-ethinylcytidin und 5-Fluoro-2'-deoxyuridylyl-(3'-5')-3'-C-ethinylcytidin zu folgenden Ergebnissen, wobei die Daten für das 3'-5'-verknüpfte Dimer im Fettdruck oberhalb der Daten für das 5'-5'- verknüpfte Isomer aufgeführt sind.

| **In vitro Testergebnisse von** | | | |
|---|---|---|---|
| **Tumorzelllinie** | | **TGI in mol/l** | **LC₅₀ in mol/l** |
| **Lunge** | | | |
| **A549/ATCC** | | **2,96 E-5** | **> 1,00 E-4** |
| | | > 1,00 E-4 | > 1,00 E-4 |
| | | | |
| **EKVX** | | **6,37 E-6** | > **1,00 E-4** |
| | | > 1,00 E-4 | > 1,00 E-4 |
| | | | |
| **HOP-92** | | **2,18 E-6** | > **1,00 E-4** |
| | | 2,15 E-5 | > 1,00 E-4 |
| | | | |
| **NCI-H522** | | **1,15 E-6** | **> 1,00 E-4** |
| | | > 1,00 E-4 | > 1,00 E-4 |
| | | | |
| **Darm** | | | |
| **HCC-2998** | | **1,09 E-7** | **1,73 E-6** |
| | | 2,95 E-6 | 5,72 E-5 |
| | | | |
| **HCT-15** | | **1,51 E-5** | **> 1,00 E-4** |
| | | > 1,00 E-4 | > 1,00 E-4 |
| | | | |
| **Zentralnervensystem** | | | |
| **SF-295** | | **1,07 E-6** | **> 1,00 E-4** |
| | | 2,06 E-5 | > 1,00 E-4 |
| | | | |
| **SF-539** | | **1,67 E-7** | **3,22 E-5** |
| | | 2,01 E-6 | > 1,00 E-4 |
| | | | |
| **SNB-75** | | **8,82 E-7** | **4,72 E-6** |
| | | > 1,00 E-4 | > 1,00 E-4 |
| | | | |
| **U251** | | **> 1,00 E-4** | > **1,00 E-4** |
| | | > 1,00 E-4 | > 1,00 E-4 |
| | | | |
| **Melanom** | | | |
| **LOX IMVI** | | **3,48 E-7** | **9,15 E-5** |
| | | 1,00 E-4 | 1,00 E-4 |
| | | | |
| **SK-MEL-2** | | **4,24 E-7** | **6.95 E-5** |
| | | 5,78 E-6 | 7,64 E-5 |
| | | | |
| **Ovarien** | | | |
| **IGROV1** | | **1,29 E-5** | **> 1,00 E-4** |
| | | > 1,00 E-4 | > 1,00 E-4 |
| | | | |
| **Niere** | | | |
| **ACHN** | | **1,52 E-6** | **> 1,00 E-4** |
| | | > 1,00 E-4 | > 1,00 E-4 |
| | | | |
| **CAK-1** | | **8,29 E-8** | **> 1,00 E-4** |
| | | > 1,00 E-4 | > 1,00 E-4 |
| | | | |
| **Prostata** | | | |
| **DU-145** | | **4,40 E-7** | **> 1,00 E-4** |
| | | > 1,00 E-4 | > 1,00 E-4 |
| | | | |
| **Brust** | | | |
| **MCF7** | | **1,37 E-7** | **> 1,00 E-4** |
| | | > 1,00 E-4 | > 1,00 E-4 |
| | | | |
| **BT-549** | | **3,46 E-7** | **6,39 E-6** |
| | | 1,16 E-5 | > 1,00 E-4 |
| | | | |
| **T-47D** | | **2,67 E-5** | **> 1,00 E-4** |
| | | > 1, 00 E-4 | > 1,00 E-4 |

## Patentansprüche

1. Ethinylierte Heterodinucleosidphosphatanaloga der Formel I 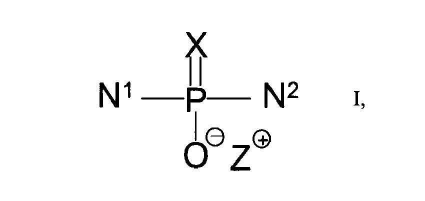 worin
X für O oder S steht;
Z für H oder das korrespondierende Säureadditionssalz dieser Verbindung steht;
N¹ und N² verschieden sind und jeweils für eine Nucleosidgruppe stehen, wobei jede der Nucleosidgruppen, die jeweils einen glycosidischen (oder davon abgeleiteten cyclischen) Rest und einen damit kovalent verknüpften Basenrest aufweisen, über deren glycosidischen Rest mit dem zentralen P-Atom kovalent verbunden (sauerstoff- oder schwefelverknüpft) ist; und wobei wenigstens eine der Nucleosidgruppen einen ethinylierten glycosidischen Rest aufweist.

2. Verbindungen nach Anspruch 1, wobei jeder der gleichen oder verschiedenen, gegebenenfalls ethinylierten glycosidischen Reste von N¹ und N² abgeleitet ist von einer Furanose, Pentose, Hexose oder Heptose, wobei ein oder mehrere ringgebundene H-Atome oder Hydroxylgruppen gegebenenfalls eliminiert oder substituiert sind durch H, Halogen, Hydroxyl, Cyano, 2-Fruormethylen, Trifluoromethyl oder Azido; gegebenenfalls ein Heteroatom, ausgewählt unter S, N und O anstelle eines Ring-Kohlenstoffatoms im glycosidischen Rest enthalten sein kann; und der glycosidische Rest gegebenenfalls eine oder zwei nichtbenachbarte C=C-Doppelbindungen enthalten kann.

3. Verbindungen nach Anspruch 1 oder 2, wobei jeder der gleichen oder verschiedenen Basenreste der Rest einer ein- oder zweikernigen heterocyclischen Base ist, die aus einem oder zwei vier- bis siebengliedrigen Ringen aufgebaut ist, wobei der Basenrest wenigstens ein basisches Ring-N-atom und gegebenenfalls wenigstens eine basische Aminogruppe und gegebenenfalls wenigstens ein weiteres Ring-Heteroatom, ausgewählt unter S und O, enthalt; und wobei der Basenrest gegebenenfalls ein- oder mehrfach substituiert ist durch Hydroxyl, Amino, Halogen, Alkyl, Alkenyl, Polyoxyalkenyl, Aryl, Acyl, Alkyloxy, Alkenyloxy, Polyoxyalkenyloxy, Acyloxy, Aryloxy, Alkylthio, Alkenylthio, Acylthio oder Arylthio; wobei die Amino-, Alkyl-, Alkenyl- und Acylreste gegebenenfalls mit 1 bis 3 Aryl-Resten, Polyoxyalkylenresten oder Halogenatomen substituiert sind.

4. Verbindungen nach einem der vorhergehenden Ansprüche, wobei N¹ und N² über gleiche oder verschiedene Positionen der glycosidischen Gruppen P-verknüpft sind.

5. Verbindungen nach einem der vorhergehenden Ansprüche, wobei jeder der gleichen oder verschiedenen glycosidischen Reste ein furanosidischer Rest oder ein davon abgeleiteter, fünfgliedriger Rest ist.

6. Verbindungen nach Anspruch 5, wobei N¹ und N² über das P-Atom 3'-5' oder 5'-5' miteinander verknüpft sind.

7. Verbindungen nach einem der vorhergehenden Ansprüche, worin X und Z die oben angegebenen Bedeutungen besitzen, Y Sauerstoff oder Schwefel bedeuten und die Gruppen N¹ und N² voneinander verschieden sind und für ein D- oder L- konfiguriertes Nucleosidderivat der Formel II, III, und IV 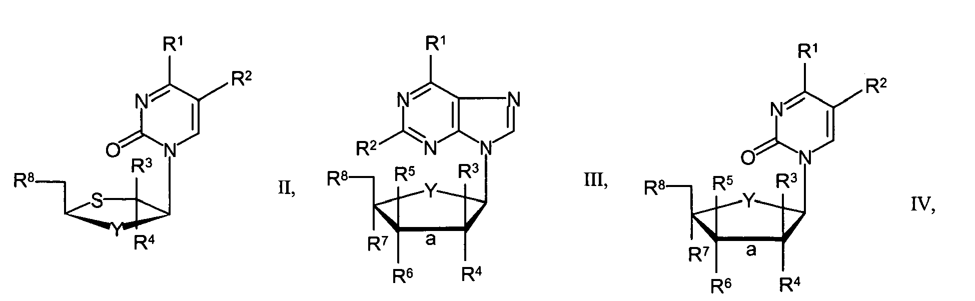 stehen, worin
Y für O oder S steht;
R¹ eine Hydroxyl-, Alkoxy-, Amino-, acylierte, alkylierte oder durch Polyoxyethylen substituierte Aminogruppe bedeutet, deren Acyl- oder Alkylrest linear oder verzweigt ist, 1 bis 24 C-Atome und bis zu 2 Doppelbindungen aufweist und durch 1 - 3 aromatische Reste oder einen Heterozyclus substituiert sein kann,
R² für H, Halogen, eine Amino-, Hydroxyl- oder Trifluormethylgruppe, einen Bromvinyl-, einen linearen oder verzweigten C₁C₂₄ Alkylrest steht;
R³ bis R⁸ gleich oder verschieden sind, und für H, Halogen, Hydroxyl, Ethinyl, Cyano, Fluoromethylen, Trifluoromethyl oder Azido stehen, wobei zwei der Reste R³ bis R⁶ dann entfallen, wenn die C-C- Bindung in Position a für eine Doppelbindung steht;
wobei N¹ und N² so gewählt sind, das immer einer der Reste R³ bis R⁸ von N¹ und N² unabhängig voneinander für -O- oder -S- steht, worüber N¹ und N² mit dem zentralen P-Atom der Formel 1 verknüpft sind; und mindestens einer der verbleibenden Reste R³ bis R⁸ in N¹ oder N² Ethinyl bedeutet, so dass entweder N¹ oder N² mindestens einen Ethinylrest aufweist.

8. Verbindungen nach Anspruch 7, worin X, Y, Z und a die oben angegebenen Bedeutungen besitzen und
a) in den Gruppen N¹ und N² unabhängig voneinander
R¹ für eine alkylierte oder acylierte Aminogruppe steht, worin deren Alkylrest ein Hexadecylrest und der Acylrest ein Palmitoyl, Oleoyl- oder Behenoylrest ist;
R² für H, Halogen, Methyl, Ethyl oder Trifluormethyl steht;
R³, R⁴ und R⁷ für Azido, H, Fluor, Fluoromethylen, Cyano, Trifluoromethyl oder Hydroxyl steht;
und gleichzeitig
b) in einer der Gruppen N¹ und N²
R⁵ für Ethinyl steht und in der anderen der Gruppen R⁵, falls vorhanden, für Azido, H, Fluor oder Hydroxyl steht;
und gleichzeitig
c) in jeder der Gruppen N¹ und N² unabhängig voneinander
einer der Reste R⁶ und R⁸ für -O- oder -S- steht und der andere der beiden Reste R⁶ und R⁸ für Azido, H, Fluor oder Hydroxyl steht.

9. Verbindungen nach einem der Ansprüche 7 und 8,
worin Z und a die oben angegebenen Bedeutungen besitzen
X für O steht;
N¹ und N² verschieden sind und für ein Nucleosidderivat der Formel IV stehen,
worin Y für O steht Sauerstoff;
R¹ für eine Amino-, C₁₂-C₂₂-Alkylamino-, C₁₂-C₂₂-Acyl-Aminogruppe oder eine Hydroxylgruppe steht;
R² für H, Fluor oder Trifluormethyl steht; und
R³ bis R⁸ die oben angegebenen Bedeutungen besitzen.

10. Verbindungen nach einem der Ansprüche 7 bis 9, worin N¹ für einen gegebenenfalls ethinylierten Nucleosidrest der Formel IV steht, worin die Reste R¹ bis R⁸ in die oben angegebenen Bedeutungen besitzen und N² für einen Nukleosidrest der Formel IV steht der ethinyliert ist, worin
R¹ für Amino
R²,R³,R⁷ für H;
R⁴, R⁶ für Hydroxyl;
R⁵ für Ethinyl; und
R⁸ für ein Sauerstoffatom steht, mit dem N² verbrückt ist.

11. Verbindungen nach Anspruch 10, worin N² für einen Nukleosidrest der Formel IV steht der ethinyliert ist, und N¹ für einen Nukleosidrest der Formel IV steht, der nicht ethinyliert ist, worin
R¹ für Hexadecyl-, Palmitoyl-, Oleoylamino oder Hydroxyl steht;
R² für H oder Fluor steht;
R³, R⁴ gleich oder verschieden sind und für H, Hydroxyl, Fluor steht;
R⁵, R⁷ für H steht;
R⁶ und R⁸ unabhängig voneinander für Hydroxyl, Azido oder H stehen, mit der Maßgabe, dass einer der Reste für -O- steht.

12. Verbindungen nach Anspruch 11, worin in N² für einen Nukleosidrest der Formel IV steht der ethinyliert ist, und N¹ für einen Nukleosidrest der Formel IV steht, der nicht ethinyliert ist, worin
R¹ für Hydroxyl steht;
R² für Fluor steht;
R³, R⁴, R⁷ für H stehen,
R⁶ für -O- steht, und
R⁸ für Hydroxyl steht .

13. Verbindungen nach einem der vorhergehenden Ansprüche, ausgewählt unter
(a) 5-Fluoro-2'-desoxyuridylyl-(3'-5')-3'-C-ethinylcytidin
(b) Arabinocytidylyl-(5'- 5')-3'-C-ethinytcytidin
(c) N⁴-Hexadecylarabinocytidylyl-(5'- 5')-3'-C-ethinylcytidin
(d) (E)-2'-Desoxy-(2-fluoromethylen)cytidyly-(3' 5')-3'-C-ethinylcytidin
(e) β-L-Dioxolancytidylyl-(5'- 5')-3'-C-ethinylcytidin
(f) 2'-C-Cyano-2'-deoxyarabinocytidylyl-(3'-5')-3'-C-ethinylcytidin
(g) 2-Chloro-(2'-deoxy)-/fluoroarabinoadenylyl-(3'-5')-3'-C-ethinylcytidin und
(h) 2'-Desoxy-2',2'-difluorocytidylyl-(3'-5')-3'-C-ethinylcytidin

14. Pharmazeutisches Mittel, enthaltend mindestens eine Verbindung nach einem der vorhergehenden Ansprüche in einem pharmazeutisch verträglichen Träger oder Verdünnungsmittel.

15. Mittel nach Anspruch 14, enthalten in Liposomen oder Nanopartikel.

16. Mittel nach Anspruch 14 oder 15, zusätzlich enthaltend wenigstens einen weiteren pharmakologischen Wirkstoff, der zur Behandlung von Infektionskrankheiten und/oder Krebserkrankungen geeignet ist.

17. Verwendung wenigstens einer Verbindung nach einem der Ansprüche 1 bis 13 zur Herstellung eines pharmazeutischen Mittels zur Prävention und/oder Therapie von Infektionskrankheiten und/oder Krebserkrankungen.

18. Verfahren zur Herstellung von ethinylierten Heterodinucleosidphosphatanaloga nach einem der Ansprüche 1 bis 13, wobei man zwei Nucleoside der allgemeinen Formeln Va und Vb
L¹-N¹ (Va)
L²-N² (Vb)
worin
N¹ und N² wie oben definiert sind und gegebenenfalls Schutzgruppen aufweisen und
L¹ und L² am glycosidischen Rest von N¹ und N² gebundene, miteinander reaktive Gruppen darstellen, wobei eine der Gruppen L¹ und L² für eine Hydroxy- oder Mercaptogruppe und der andere für eine Hydrogenphosphonat- oder Thiohydrogenphosphonatgruppe steht;
in Gegenwart eines Säurechlorids kondensiert und das Kondensationsprodukt anschließend oxidiert, und gegebenenfalls vorhandenen Schutzgruppen entfernt.

19. Verfahren nach Anspruch 18, wobei man jeweils zwei Nucleoside der Formeln Va und Vb umsetzt, wobei die Nucleoside Va und Vb einer Verbindung der obigen allgemeinen Formel II, III oder IV entsprechen, worin
X und a die oben angegebenen Bedeutungen besitzen,
L¹ und L² an Stelle eines der Reste R³ bis R⁸ enthalten sind,
die Reste R¹ bis R⁸ im übrigen die in den Ansprüchen 7 bis 17 angegebene Bedeutung besitzen;
die Reste R¹ und R³ bis R⁸ zusätzlich auch für eine acylierte Hydroxygruppe, deren Acylrest linear oder verzweigt ist, 1-24 C-Atome und 1 oder 2 Doppelbindungen aufweist und durch einen aromatischen Rest substituiert sein kann, oder für tert-Butyldimethylsilyloxy-Schutzgruppe stehen können,
R⁸ zusätzlich auch für eine 4-Mono-, oder 4,4'-Dimethoxytriphenylmethyloxy-Schutzgruppe stehen kann;
und wenigstens einer der Reste R³ bis R⁸ auch für Trimethylsilylethinyl stehen kann.

20. Verfahren nach Anspruch 19, wobei man gegebenenfalls vorhandene 4-Mono- oder 4,4'- Dimethoxytriphenylmethyloxy Schutzgruppen gegen Hydroxyl tauscht und Acyl- und Silylreste gegebenenfalls hydrolytisch spaltet.
